# EUROPÄISCHE PATENTSCHRIFT

(11) **EP 3 765 594 B1**
(45) Veröffentlichungstag und Bekanntmachung des Hinweises auf die Patenterteilung: **12.07.2023**
(21) Anmeldenummer: 19710617.2
(22) Anmeldetag: 28.02.2019
(51) Int. Cl.: C12M 1/42

(54) **MIKROWELLENPORATOR**
MICROWAVE PORATOR
DISPOSITIF DE FORMATION DE PORES PAR MICRO-ONDES

(30) Priorität: 14.03.2018 DE 102018105931
(43) Veröffentlichungstag der Anmeldung: 20.01.2021
(73) Patentinhaber: Technische Universität Darmstadt, 64289 Darmstadt (DE)
(72) Erfinder: SCHMIDT, Sönke, 64289 Darmstadt (DE); SCHÜSSLER, Martin, 63768 Hösbach (DE); JAKOBY, Rolf, 61191 Rosbach (DE); CARDOSO, Maria Cristina, 64383 Darmstadt (DE); BERTULAT, Bianca, 64807 Dieburg (DE); HERCE, Henry David, 64287 Darmstadt (DE)
(74) Vertreter: LifeTech IP Spies & Behrndt Patentanwälte PartG mbB
(86) Internationale Anmeldenummer: PCT/EP2019/054983
(87) Internationale Veröffentlichungsnummer: WO 2019/174923

(56) Entgegenhaltungen:
- US-A1- 2007 179 535
- US-A1- 2010 219 076
- US-A1- 2013 108 667
- US-B1- 6 699 698

## Beschreibung

Die vorliegende Erfindung bezieht sich auf einen Mikrowellenporator und ein Verfahren zur Behandlung von biologischen Substanzen, insbesondere zum Einbringen von Makromolekülen in biologischen Zellen.

### Hintergrund

Auf folgende Dokumente wird im Kontext der Mikrowellen-Poration der biologischen Zellen verwiesen: US2013/0108667 A1, US2007/0179535 A1 und US 6,699,698 B1.

Es hat sich gezeigt, dass biologische Zellen bei einer Behandlung mit einer Mikrowellenstrahlung durchlässig zum Beispiel für Proteine werden. Ein Mikrowellenporator nutzt diesen Effekt, um mittels eines Mikrowellenfeldes biologische Substanzen zu behandeln und insbesondere um Zellmembranen durchlässig für Makromoleküle wie beispielsweise ein Protein zu machen. Dieser Vorgang soll in einer kontrollierten und beobachtbaren Weise durchgeführt werden. US 6,190,380 B1 offenbart beispielhaft eine Zellen-Transfektionsvorrichtung, die ein Mikrowellenstrahlungsfeld innerhalb eines Patienten dazu nutzt, um DNA in die Zellen einzuschleusen.

Nachteile von bekannten Systemen bestehen darin, dass diese nur eine unzureichende In situ-Beobachtung des Erfolges der Behandlung ermöglichen und dass die Ausbeute, d. h. die Überlebensrate der behandelten Zellen, noch zu gering ist.

Daher besteht ein Bedarf nach Mikrowellenporatoren, die die genannten Probleme überwinden und insbesondere zur Behandlung von biologischen Substanzen wie Säugerzellen geeignet sind.

### Kurzbeschreibung der Erfindung

Zumindest ein Teil der obengenannten Probleme wird durch einen Mikrowellenporator Anspruch 1 und ein Verfahren Anspruch 12 gelöst. Die abhängigen Ansprüche definieren weitere vorteilhafte Ausführungsformen für den Mikrowellenporator.

Die vorliegende Erfindung bezieht sich auf einen Mikrowellenporator zum Einbringen von Makromolekülen in biologischen Zellen. Der Mikrowellenporator umfasst: eine Zuleitung für ein Mikrowellensignal; einen Behälter für die biologischen Zellen und die Makromoleküle und eine Elektrodenstruktur mit zumindest zwei Elektroden. Die zumindest zwei Elektroden sind zumindest teilweise in dem Behälter ausgebildet und mit der Zuleitung verbunden oder verbindbar, um die biologischen Zellen dem Mikrowellenfeld auszusetzen. Die Elektrodenstruktur erstreckt sich entlang eines Randes oder in einen Innenraum des Behälters und weist eine Form auf, so dass sich zwischen den zumindest zwei Elektroden ein inhomogenes Mikrowellenfeld bildet. In einer Ausführungsform ist zwischen den zumindest zwei Elektroden ein Spalt gebildet, der sich mäanderförmig erstreckt oder so gebildet ist, dass die erste Elektrode innerhalb der zweiten Elektrode in eine Spitze monoton zulaufend ausgebildet ist. Alternativ erstrecken sich die zumindest zwei Elektroden in den Innenraum des Behälters, um in dem Innenraum durch die biologischen Zellen und/oder die Makromoleküle umschlossen zu sein.

Optional ist zwischen den zumindest zwei Elektroden ein Spalt gebildet, der eine mittlere Breite in einem Bereich zwischen 10 µm und 500 µm oder zwischen 50 µm und 100 µm aufweist und sich mäanderförmig erstreckt (d.h. die Richtung der Feldlinien ändern sich entlang des Spaltes fortlaufend in verschiedene Richtungen). Die Abmaße und die Form des Spaltes können beispielsweise entlang der Feldlinien des Mikrowellenfeldes zwischen den Elektroden definiert werden. Die Elektroden können zum Beispiel auf eine Wand des Innenraumes des Behälters aufgedampft werden, wobei beispielsweise unter Nutzung von fotolithografischen Verfahren besonders kleine Strukturen herstellbar sind. Der Begriff "inhomogenes Mikrowellenfeld" soll insbesondere ein Mikrowellenfeld umfassen, welches vom Ort abhängt und daher nicht translationssymmetrisch ist. Ein Feld zwischen zwei parallelen Platten wäre beispielsweise ein homogenes Feld, während der sich beispielhaft mäanderförmig erstreckende Spalt zu sich ändernden Feldrichtungen oder zu Bereichen hoher und niedrigerer Feldstärken führt. Außerdem wird in Spaltnähe die Feldstärke sehr groß sein, während sie mit größer werdendem Abstand von dem Spalt sich verringert. Ein Vorteil des mäanderförmigen Spaltes liegt darin, dass dadurch eine große Fläche mit hohen Feldstärken vorliegt und so die Effizienz der Mikrowellenporation erhöht wird.

Optional kann die Form des Spaltes zwischen den zumindest zwei Elektroden derart gewählt werden, dass Reflexionen bei einem Einspeisen des Mikrowellenfeldes unterdrückt werden. Dies ist gleichbedeutend dazu, dass die Absorption (oder der Durchlass) maximiert wird. Als Folge steht eine maximale Leistung für den Porationseffekt zur Verfügung. Dies kann beispielsweise durch eine Impedanzanpassung beim Einleiten der Mikrowellenstrahlung in die biologischen Zellen erfolgen, die wiederum dadurch erreicht werden kann, dass die Elektrodenstruktur eine Hochfrequenz-Anpassschaltung darstellt.

Die zumindest zwei Elektroden erstrecken sich in den Innenraum des Behälters, und zwar derart, dass sie in dem Innenraum durch die biologischen Zellen und/oder die Makromoleküle umschlossen sind (zumindest nach dem Einbringen in dem Behälter). Die Elektrodenstruktur kann demzufolge dreidimensional ausgebildet werden (nicht nur lateral oder flächenförmig). Beispielsweise kann die Elektrodenstruktur in Form eines ersten und eines zweiten Nagelbettes gebildet sein, sodass Nägel des ersten und zweiten Nagelbettes benachbart zueinander angeordnet oder ineinander schiebbar sind. Das Mikrowellenfeld wird dann zwischen den einzelnen Nägeln der Nagelbetten herausgebildet.

Optional weist der Behälter einen verschiebbaren Stempel auf. Eine der zumindest zwei Elektroden ist an einem Randbereich des Behälters ausgebildet und die andere der zumindest zwei Elektroden ist an dem Stempel derart ausgebildet, dass bei einer Verschiebung des Stempels ein Abstand zwischen den beiden Elektroden sich ändert. Die biologischen Zellen sind zumindest teilweise zwischen dem Stempel und dem Randbereich einbringbar.

Optional umfasst der Behälter ein Fenster, um eine optische Analyse (z.B. durch eine Bilderfassung mittels eines Mikroskops) über einen Fortschritt bei der Behandlung der biologischen Zellen zu ermöglichen. Das Fenster kann beispielsweise aus Glas gebildet sein. Es ist auch möglich, dass der gesamte Behälter - bis auf die Elektrodenstruktur - aus einem Glas gebildet ist.

Optional umfasst der Behälter einen Temperatursensor, um eine Temperaturerfassung der biologischen Zellen während der Behandlung zu ermöglichen.

Optional umfasst der Mikrowellenporator weiter eine Steuereinheit, die ausgebildet ist, um das Mikrowellenfeld an der Zuleitung anzulegen oder zur Ermittlung eines Erfolgs der Behandlung eine Impedanzspektroskopie durchzuführen. Bei der Impedanzspektroskopie erfolgt mittels eines Sensorwechselfelds eine Impedanzmessung der biologischen Zellen. Da diese Impedanz für porierte Zellen anders ist als für nichtporierte Zellen, kann hierdurch eine Überwachung der Poration durchgeführt werden, ohne dass eine optische Untersuchung notwendig ist. Optional ist die Steuereinheit ausgebildet, um die Impedanzspektroskopie unter Nutzung der (vorhandenen) Elektrodenstruktur oder unter Nutzung weiterer Messelektroden durchzuführen.

Optional erstreckt sich die Elektrodenstruktur durch eine Behälterwand hindurch. An einem Außenbereich des Behälters können dann Kontakte an der Elektrodenstruktur vorhanden sein, um die Zuleitung daran zu kontaktieren.

Optional ist der Behälter Teil einer Vielzahl von Behältern, die verschiebbar zueinander angeordnet sind und jeweils die zumindest zwei Elektroden aufweisen, sodass durch eine Verschiebung der Behälter die Zuleitung mit den jeweiligen Elektroden der Behälter kontaktiert wird. Damit wird eine fortlaufende Behandlung von biologischen Zellen in den einzelnen Behältern durch eine Verschiebung der Behälter ermöglicht.

Die vorliegende Erfindung bezieht sich auch auf ein Verfahren zur Behandlung von biologischen Zellen unter Nutzung eines zuvor beschriebenen Mikrowellenporators.

Ausführungsbeispiele der vorliegenden Erfindung überwinden zumindest einen Teil der eingangs erwähnten Nachteile durch eine spezielle Elektrodenstruktur, die in dem Behälter ausgebildet ist, um ausreichend hohe Feldstärken anwenden zu können, ohne dass es zu ungewünschten Reflektionen beim Zuleiten des Mikrowellenfeldes kommt.

Damit bieten Ausführungsbeispiele im Vergleich zum Stand der Technik die folgenden Vorteile:
- Die Mikrowellenporation kann direkt auf Zellkulturen angewendet werden. Das Überführen der Zelle in ein anderes Gefäß ist nicht erforderlich. Insbesondere ist kein Ablösen von adhärenten Zellen erforderlich.
- Zellen können durchgehend mit einem Mikroskop beobachtet werden oder optional mittels Impedanzspektroskopie analysiert werden.
- Eine höhere Ausbeute (d.h. eine höhere Überlebensrate der behandelten Zellen) ist gewährleistet. Ebenso können auch große Moleküle durch die Zellen aufgenommen werden.

Diese Vorteile werden insbesondere durch deutlich niedrigere Feldstärken erreicht (z. B.: um einen Faktor von 100 bis zu 1000 kleiner als bei dem zuvor genannten Stand der Technik). Die genutzte Elektrodenstruktur ist besonders für die Mikrowellenfrequenzen in einem Frequenzbereich zwischen 10 und 30 GHz oder in einem Bereich zwischen 15 und 20 GHz angepasst (oder für die 18 GHz-Mikrowellenporation). Es hat sich gezeigt, dass gerade diese Frequenzen den gewünschten Effekt bieten.

### Kurzbeschreibung der Figuren

Die Ausführungsbeispiele der vorliegenden Erfindung werden besser verstanden anhand der folgenden detaillierten Beschreibung und den beiliegenden Zeichnungen der unterschiedlichen Ausführungsbeispiele, die jedoch nicht so verstanden werden sollten, dass sie die Offenbarung auf die spezifischen Ausführungsformen einschränken, sondern lediglich der Erklärung und dem Verständnis dienen.
- Fig. 1: zeigt einen Mikrowellenporator gemäß einem Ausführungsbeispiel der vorliegenden Erfindung.
- Fig. 2: zeigt mögliche Ausgestaltungen der Elektrodenstruktur.
- Fig. 3A-3C: zeigen weitere Ausgestaltungsmöglichkeiten für die Elektrodenstruktur.
- Fig. 4: zeigt ein System mit einem Mikrowellenporator gemäß weiterer Ausführungsbeispiele.
- Fig. 5: zeigt ein Ausführungsbeispiel für einen Mikrowellenporator mit einem optischen Fenster, um beispielsweise eine Analyse mittels Mikroskop zu erlauben.
- Fig. 6: zeigt Ausführungsbeispiel für eine Mikrowellen-Assay-Anordnung mit mehreren Behältern.

### Detaillierte Beschreibung

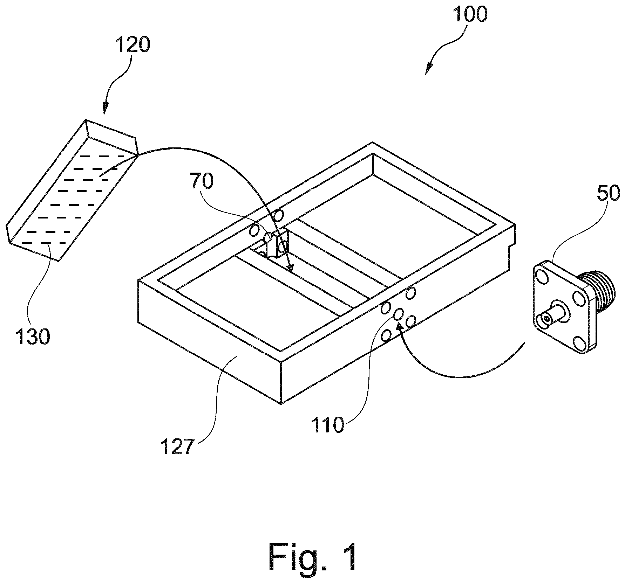

**Fig. 1** zeigt beispielhaft einen Mikrowellenporator 100, der geeignet ist, um Makromoleküle (z.B. Proteine, DNA) in biologische Zellen einzubringen. Der Mikrowellenporator 100 umfasst: eine Zuleitung 110 für ein Mikrowellenfeld, einen Behälter 120 und eine Elektrodenstruktur 130 mit zumindest zwei Elektroden (in der Fig. 1 nicht zu sehen), die zumindest teilweise in dem Behälter 120 ausgebildet und mit der Zuleitung 110 verbindbar sind, um biologische Zellen in dem Behälter 120 dem Mikrowellenfeld an der Zuleitung 110 auszusetzen. Die Elektrodenstruktur 130 erstreckt sich entlang eines Randes des Behälters 120 und weist eine Form auf, so dass sich zwischen den zumindest zwei Elektroden ein inhomogenes Mikrowellenfeld bildet.

Der gezeigten Mikrowellenporator 100 umfasst einen optionalen Steckanschluss 50 für ein Hochfrequenzkabel, welches beispielsweise aufgeschraubt werden kann. Ebenso umfasst der Mikrowellenporator 100 beispielhaft eine Nut 70 für eine Masseverbindung, so dass der Steckanschluss 50 das Signal zuführt und der Masseanschluss über die Nut 70 bereitgestellt wird. Der gezeigte Behälter 120 umfasst an seiner Unterseite die Elektrodenstruktur 130 und kann in einer Aussparung einer Halterung 127 eingesetzt werden. Nach dem Einsetzen kann automatisch eine elektrische Verbindung zu dem Steckanschluss 50 und zur Masse hergestellt sein.

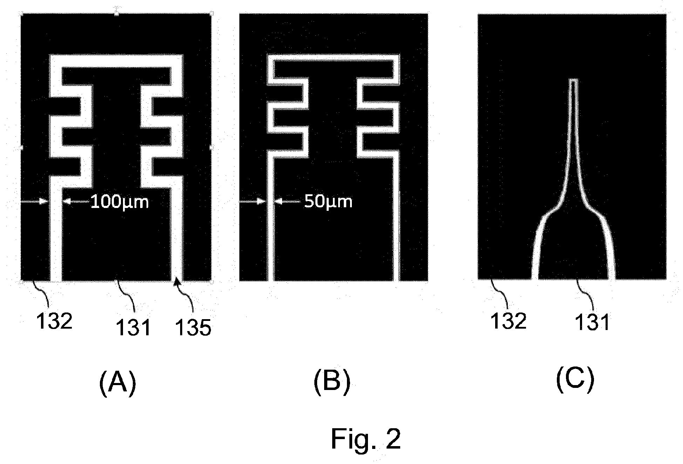

**Fig. 2** zeigt mögliche Ausgestaltungen der Elektrodenstruktur 130. Die Elektrodenstruktur 130 umfasst beispielhaft eine erste Elektrode 131 und eine zweite Elektrode 132. Eine der beiden Elektroden (z.B. die zweite Elektroden 132) kann mit dem Masseanschluss verbunden sein und die andere, erste Elektrode 131 ist mit der Zuleitung 110 verbunden, um das Mikrowellensignal einzuspeisen. Zwischen der ersten Elektrode 131 und der zweiten Elektrode 132 ist ein Spalt 135 ausgebildet. Der Spalt 135 kann beispielsweise eine Breite in einem Bereich zwischen 10 und 500 µm aufweisen.

In der Elektrodenstruktur (A) ist der Spalt beispielhaft ca. 100 µm und in der Elektrodenstruktur (B) ist der Spalt beispielhaft ca. 50 µm breit. Der Spalt 135 erstreckt sich in den beiden Ausführungsformen (A) und (B) mäanderförmig entlang der Elektrodenstruktur 130 und trennt elektrisch die erste Elektrode 131 von der zweiten Elektrode 132, die zumindest teilweise die erste Elektrode umschließt.

Es ist ebenfalls möglich, siehe Ausgestaltung (C), dass die erste Elektrode 131 innerhalb der zweiten Elektrode 132 in eine Spitze monoton zulaufend ausgebildet ist, wobei wiederum ein Spalt mit einer vorbestimmten Breite (z.B. zwischen 10 µm und 500 µm) zwischen der ersten Elektrode 131 und der zweiten Elektrode 132 ausgebildet ist. Die spitz zulaufende erste Elektrode 131 bietet den Vorteil, dass beim Einspeisen des Hochfrequenzsignals keine Impedanzsprünge auftreten und daher Reflektionen unterdrückt werden. Die eingespeiste Energie wird effizient an die biologische Substanz weitergegeben und zur Poration genutzt.

Es versteht sich, dass die gezeigte Anpassschaltung (C) für die Elektrodenstruktur 130 lediglich ein Beispiel darstellt. Die vorliegende Erfindung soll nicht auf diese besondere Elektrodenstruktur eingeschränkt sein. Insbesondere ist jede Elektrodenstruktur 130 verwendbar, die eine Anpassschaltung umsetzt, so dass für die genutzte Mikrowellenstrahlung Reflektionen weitestgehend unterdrückt werden.

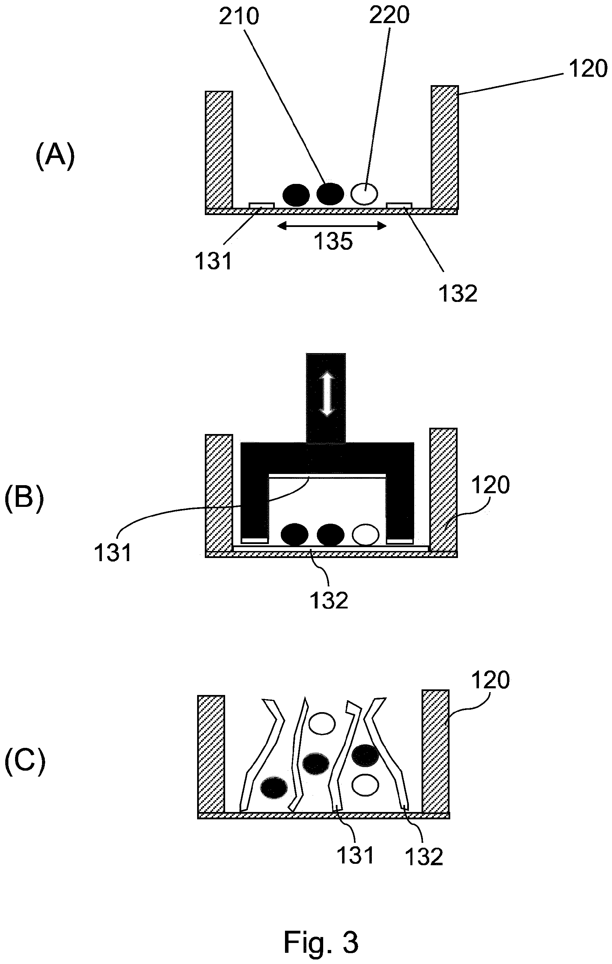

**Fig. 3A-3C** zeigen weitere Ausgestaltungsmöglichkeiten für die erste und zweite Elektrode 131, 132 als Teil der Elektrodenstruktur 130. Die Ausführungen gemäß Fig. 3A und 3B sind nicht Bestandteil der vorliegenden Erfindung.

In der Fig. 3A sind die beiden Elektroden 131, 132 in einem Bodenbereich des Behälters 120 flächenförmig ausgebildet, so dass in dem Spalt 135 zwischen den beiden Elektroden 131, 132 porierte Zellen 210 (sogenanntes Uptakes) und nichtporierte Zellen 220 vorhanden sind. Die porierten Zellen 210 haben bereits die Makromoleküle aufgenommen, während die nichtporierten Zellen 220 die Makromoleküle noch nicht aus der Umgebung aufgenommen haben.

Die Fig. 3B zeigt ein weiteres Ausführungsbeispiel, bei welchem der Behälter 120 einen Stempel 140 mit einer Elektrode 131, 132 aufweist und auf dem Bodenbereich beispielhaft die andere Elektrode 132, 131 ausgebildet ist. Der Stempel 140 ist in vertikaler Richtung bewegbar, sodass während der Bewegung des Stempels 140 der Abstand zwischen den beiden Elektroden 131, 132 sich ändert. Damit wird die Feldstärke, die auf Zellen 210, 220 wirkt, einstellbar und der Effekt der Poration kann verbessert und optimiert werden.

Die Fig. 3C zeigt ein Beispiel, bei welchem die Elektroden 131, 132 nicht planar, sondern dreidimensional ausgebildet sind. Sie erstrecken sich in dem Innenraum des Behälters 120 hinein, sodass die Elektroden 131, 132 von beiden Seiten durch die porierten Zellen 210 und nichtporierten Zellen 220 umströmt werden können.

Die konkrete Form der sich in dem Behälter hineinerstreckenden Elektroden 131, 132 kann weitestgehend beliebig gewählt werden, wobei es wiederum vorteilhaft ist, wenn die Elektrodenform so gewählt ist, dass sich eine Impedanzanpassung ergibt (um einen effektiven Energieübertrag zu gewährleisten).

So ist es beispielsweise ebenfalls möglich, dass die ersten Elektroden 131 ein Nagelbett darstellen und die zweiten Elektroden 132 ebenfalls ein Nagelbett darstellen. Die Vielzahl von Nägeln aus dem ersten Nagelbett können so ausgebildet werden, dass sie sich mit den Nägeln aus dem zweiten Nagelbett abwechseln, um eine möglichst hohe Feldstärke im gesamten Innenraum zu erreichen. Dies führt wiederum zu einer Intensivierung des Porationseffektes. Beispielhaft können die Nägel in dergleichen Weise ausgebildet werden, wie es die Elektrodenform aus der Fig. 2C zeigt.

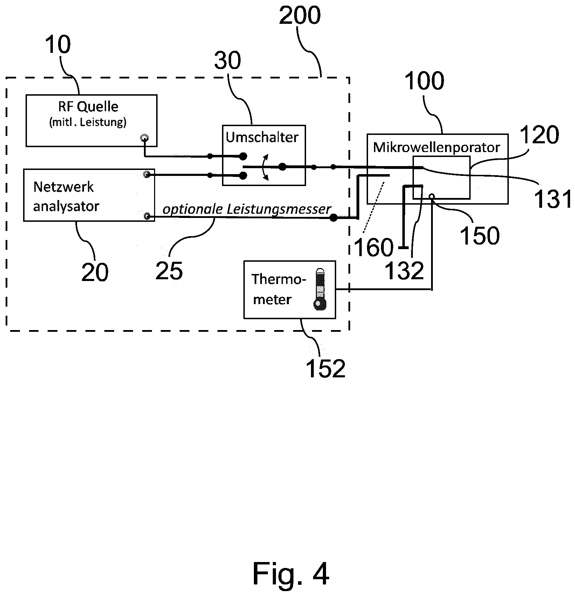

**Fig. 4** zeigt beispielhaft ein System gemäß weiterer Ausführungsbeispiele. Das System umfasst eine Hochfrequenzquelle 10, einen Umschalter 30, einen Netzwerkanalysator 20 und einen der zuvor beschriebenen Mikrowellenporatoren 100. Außerdem umfasst das System einen Temperatursensor 150, der mit einer entsprechenden Temperaturerfassungseinheit 152 verbunden ist, um eine Temperaturüberwachung innerhalb des Behälters 120 durchzuführen. Abgesehen von dem Mikrowellenporator 100 können alle oder einige der anderen Komponenten Teil einer Steuereinheit 200 sein.

Die Hochfrequenzquelle 10 erzeugt das Mikrowellenfeldsignal. Der Umschalter 30 ist ausgebildet, um zwischen dem Hochfrequenzsignal von der Hochfrequenzquelle 10 und einem Analysesignal von dem Netzwerkanalysator 20 umzuschalten. Der Netzwerkanalysator 20 ist ausgebildet, um ein Analysesignal bereitzustellen, welches zum Beispiel eine Impedanzmessung in dem Behälter 120 des Mikrowellenporators 100 vornimmt. Zusätzlich kann der Netzwerkanalysator (oder ein optionaler Leistungsmesser) kontinuierlich dazu genutzt werden, die Feldstärke mit einem Richtkoppler 160 an den Elektroden während des Porationsvorgangs zu überwachen. Diese Massnahme dient zur Verbesserung der Reproduzierbarkeit der Porationsvorgänge. Die erste Elektrode 131 ist mit der Zuleitung 110 verbunden, über welche die Mikrowellenstrahlung von der Hochfrequenzquelle 10 in den Mikrowellenporator 100 gelangt. Nach dem Umschalten kann auch das Analysesignal von dem Netzwerkanalysator 20 zu der ersten Elektrode 131 geführt werden.

Mit dem gezeigten System ist es möglich, eine Impedanzspektroskopie durchzuführen, bei welcher die Impedanz der biologischen Substanz gemessen wird. Dazu stellt der Netzwerkanalysator 30 ein vorbestimmtes Analysesignal bereit, welches geeignet ist, um die Impedanz der biologischen Substanz zu messen. Da die Mikrowellenporation dazu führt, dass die Impedanz der biologischen Substanz sich ändert, kann der Erfolg der Behandlung mittels dieser Impedanzspektroskopie ermittelt werden. Für die Impedanzspektroskopie kann einerseits die erste Elektrode 131 über den Umschalter 30 genutzt werden. Alternativ oder in Kombination dazu ist es ebenfalls möglich, eine separate Leitung 25 zu nutzen, die an einem vorbestimmten Punkt innerhalb des Mikrowellenporators 100 ein vorbestimmtes elektromagnetisches Signal einspeist. Dazu kann beispielsweise ein direktiver Koppler 160 genutzt werden, der derart angeordnet ist, dass das Analysesignal in eine gewünschte Richtung in den Behälter 120 eingespeist wird.

Diese Ausführungsform bietet besonders den Vorteil, dass keine optische Analyse erforderlich ist, da die Impedanzspektroskopie bereits die gewünschten Ergebnisse liefert.

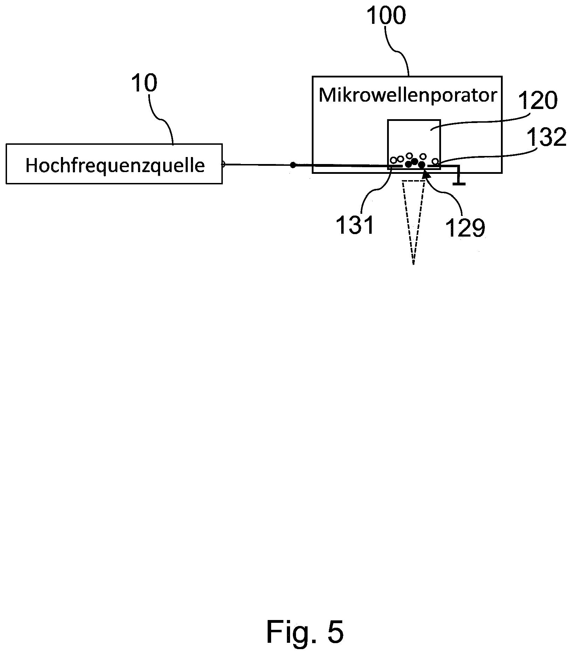

**Fig. 5** zeigt ein weiteres Ausführungsbeispiel, bei welchem der Mikrowellenporator 100 ein optisches Fenster 129 aufweist, um eine optische Analyse des Porationserfolges (z.B. mittels eines Mikroskops) durchzuführen. Das Fenster 129 kann ein Glasbereich sein, der sich zumindest zwischen den beiden Elektroden 131, 132 befindet, um die Anzahl der porierten Zellen festzustellen. Auf diese Weise braucht kein zusätzlicher Netzwerkanalysator 20 und Umschalter 30 aus der Fig. 4 vorhanden sein. Das System weist lediglich eine Hochfrequenzquelle 10 auf, die über die erste Elektrode 131 eine Mikrowellenstrahlung anlegt. Die zweite Elektrode 132 ist beispielsweise mit einem Masseanschluss verbunden.

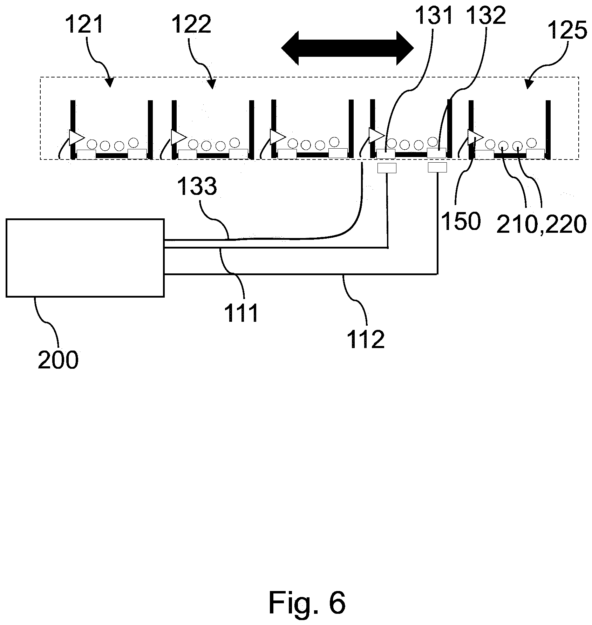

**Fig. 6** zeigt beispielhaft ein Mikrowellen-Assay-Anordnung, die einen hohen Durchsatz zulässt und eine schnelle und automatisierte Prozessierung einer Vielzahl von Porationskammern 121, 122, ... (Behälter 120) erlaubt. Beispielshaft umfasst das System fünf Porationskammern 121, 122, ..., 125, die jeweils eine erste Elektrode 131 und eine zweite Elektrode 132 aufweisen, zwischen denen sich Zellen 210, 220 befinden.

Die Porationskammern 121, ..., 125 sind in der Fig. 6 horizontal beweglich, so dass die ersten Elektroden 131, 132 in den einzelnen Porationskammern 121, 122, ... nacheinander mit einer ersten Zuleitung 111 und einer zweiten Zuleitung 112 verbindbar sind. Die erste Zuleitung 111 und die zweite Zuleitung 112 ist mit einer Steuereinheit 200 verbunden. Die Steuereinheit 200 ist ebenfalls über eine Leitung 133 mit einem jeweiligen Temperatursensor 150 in den einzelnen Porationskammern 121, 122, ... verbunden.

Auf diese Weise wird es möglich, dass durch eine horizontale Verschiebung der Porationskammer 121, 122, ... nacheinander eine Mikrowellenporation in den einzelnen Porationskammern 121, 122, ... ausgelöst werden kann. Der Erfolg der Mikrowellenporation kann entweder über die zuvor genannte Impedanzspektroskopie festgestellt werden oder über eine optische Analyse, die wiederum über ein optisches Fenster (zum Beispiel mittels eines Mikroskops) durchgeführt werden kann. Die Steuereinheit 200 kann beispielsweise alle oder einige der Komponenten aufweisen, wie sie in der Fig. 4 dargestellt sind und kann wiederum ausgebildet sein, um eine Leistungsregelung durchzuführen und eine Impedanzspektroskopie durchzuführen.

In der Fig. 6 werden die Elektroden 131, 132 beispielsweise von einer Seite über Kontakte - galvanisch oder kapazitiv - mit niedrigen HF-Verlusten kontaktiert. Es ist jedoch ebenfalls möglich, dass ein Masseanschluss für alle Porationskammern 121, 122, ... fest nach unten abgeführt wird und die andere Elektrode über beweglichen Stempel 140, wie er in der Fig. 3B gezeigt ist, jeweils in die Porationskammern 121, 122, ... gebracht wird. Auf diese Weise kann der Stempel 140 nacheinander in verschiedene Porationskammern 121, 122, ... eingeführt werden. Nach dem Absenken kann die Mikrowellenstrahlung angelegt werden, um die Mikrowellenporation auszulösen. Nach einer Verschiebung der einzelnen Behälter 121, 122, ... kann auf diese Weise an einer Vielzahl von Proben eine Mikrowellenporation effizient durchgeführt werden.

Die in der Beschreibung, den Ansprüchen und den Figuren offenbarten Merkmale der Erfindung können sowohl einzeln als auch in beliebiger Kombination für die Verwirklichung der Erfindung wesentlich sein.

### Bezugszeichenliste

- 10: Hochfrequenzquelle
- 20: Netzwerkanalysator
- 25: Leitung zur Analyse
- 30: Umschalter
- 50: Steckanschluss
- 70: Nut für Masseanschluss
- 100: Mikrowellenporator
- 110, 111,: Zuleitung
- 120, 121, .: Behälter
- 127: Halterung für Behälter
- 129: Fenster
- 130: Elektrodenstruktur
- 131, 132: erste und zweite Elektroden
- 133: Temperatursignalleitung
- 135: Spalt
- 140: Stempel
- 150: Temperatursensor
- 152: Temperaturerfassungseinheit
- 160: direktiver Koppler
- 200: Steuereinheit
- 210,220: Zellen

## Patentansprüche

1. Mikrowellenporator (100) zum Einbringen von Makromolekülen in biologische Zellen (220), mit folgenden Merkmalen:
eine Zuleitung (110) für ein Mikrowellenfeld;
ein Behälter (120) für die biologischen Zellen (220) und die Makromoleküle; und
eine Elektrodenstruktur (130) mit zumindest zwei Elektroden (131, 132),
die zumindest teilweise in dem Behälter (120) ausgebildet und mit der Zuleitung (110) verbunden oder verbindbar sind, um die biologischen Zellen (220) dem Mikrowellenfeld auszusetzen,
wobei die Elektrodenstruktur (130) sich entlang eines Randes oder in einen Innenraum des Behälters erstreckt und eine Form aufweist, so dass sich zwischen den zumindest zwei Elektroden (131, 132) ein inhomogenes
Mikrowellenfeld bildet, 1) wobei zwischen den zumindest zwei Elektroden ein Spalt (135) gebildet ist, der 1a) sich mäanderförmig erstreckt, oder 1b) so gebildet ist, dass eine der Elektroden (131) innerhalb der anderen der Elektroden (132) in eine Spitze monoton zulaufend ausgebildet ist, oder 2) wobei die zumindest zwei Elektroden (131, 132),
zwischen denen sich das inhomogene Mikrowellenfeld bildet, sich in den Innenraum des Behälters (120) erstrecken, um in dem Innenraum durch die biologischen Zellen (220) und/oder die Makromoleküle umschlossen zu sein.

2. Mikrowellenporator (100) nach Anspruch 1, wobei der Spalt (135) eine mittlere Breite in einem Bereich zwischen 10 µm und 500 µm aufweist.

3. Mikrowellenporator (100) nach Anspruch 2, wobei der Spalt (135) zwischen den zumindest zwei Elektroden (131, 132) eine Form aufweist, um eine Impedanzanpassung beim Einleiten der Mikrowellenfeldes zu erreichen und somit Reflektionen bei einem Einspeisen des Mikrowellenfeldes zu unterdrücken.

4. Mikrowellenporator (100) nach einem der vorhergehenden Ansprüche, wobei die Elektrodenstruktur (130) in Form eines ersten und eines zweiten Nagelbettes gebildet werden, sodass Nägel des ersten und zweiten Nagelbettes benachbart zueinander angeordnet oder ineinander schiebbar sind.

5. Mikrowellenporator (100) nach einem der vorhergehenden Ansprüche, wobei der Behälter (120) einen verschiebbaren Stempel (140) aufweist und eine der zumindest zwei Elektroden (131) an einem Randbereich des Behälters ausgebildet ist und die zweite der zumindest zwei Elektroden (132) an dem Stempel (140) derart ausgebildet ist, dass bei eine Verschiebung des Stempels (140) ein Abstand zwischen den beiden Elektroden (131, 132) sich ändert und die biologischen Zellen (220) zwischen dem Stempel (140) und dem Randbereich einbringbar sind.

6. Mikrowellenporator (100) nach einem der vorhergehenden Ansprüche, wobei zumindest ein Bereich des Behälters (120) ein Fenster (129) aufweist, um eine optische Analyse über einen Fortschritt bei der Behandlung der biologischen Zellen (220) zu ermöglichen.

7. Mikrowellenporator (100) nach einem der vorhergehenden Ansprüche, wobei der Behälter (120) einen Temperatursensor (150) aufweist, um eine Temperaturerfassung der biologischen Zellen (220) während der Behandlung zu ermöglichen.

8. Mikrowellenporator (100) nach einem der vorhergehenden Ansprüche, der weiter eine Steuereinheit (200) umfasst, die ausgebildet ist, um das Mikrowellenfeld an der Zuleitung (110) anzulegen oder zur Ermittlung eines Erfolges des Einbringens der Makromoleküle in die biologischen Zellen (220) eine Impedanzspektroskopie durchzuführen, bei der mittels eines Sensorwechselfeldes eine Impedanzmessung der biologischen Zellen (220) erfolgt.

9. Mikrowellenporator (100) nach Anspruch 8, wobei die Steuereinheit (200) ausgebildet ist, um die Impedanzspektroskopie unter Nutzung der Elektrodenstruktur (130) oder unter Nutzung weiterer Messelektroden durchzuführen.

10. Mikrowellenporator (100) nach einem der vorhergehenden Ansprüche, bei dem die Elektrodenstruktur (130) sich durch eine Behälterwand erstreckt und an einem Außenbereich des Behälters (120) Kontakte aufweist, an die die Zuleitung (110) kontaktierbar sind.

11. Mikrowellenporator (100) nach einem der vorhergehenden Ansprüche, wobei der Behälter (120) Teil einer Vielzahl von Behältern (121, 122, ...) ist, die verschiebbar zueinander angeordnet sind und jeweils die zumindest zwei Elektroden (131, 132) aufweisen, sodass durch eine Verschiebung der Behälter (121, 122, ...) eine Kontaktierung der Zuleitung (110) durch die jeweiligen Elektroden in den Behältern (121, 122, ...) erfolgt.

12. Verfahren zur Behandlung von biologischen Zellen (220) unter Nutzung eines Mikrowellenporators (100) nach einem der vorhergehenden Ansprüche.

## Claims

1. Microwave porator (100) for introducing macromolecules into biological cells (220), comprising the following features:
a supply line (110) for a microwave field;
a container (120) for the biological cells (220) and the macromolecules; and
an electrode structure (130) having at least two electrodes (131, 132),
which is formed at least in part in the container (120) and is or can be connected to the supply line (110) in order to expose the biological cells (220) to the microwave field,
wherein the electrode structure (130) extends along an edge or in an interior of the container and has a shape, such that a non-homogeneous microwave field is formed between the at least two electrodes (131, 132), 1) wherein a gap (135) is formed between the at least two electrodes, which gap 1a) extends in a meandering manner, or 1b) is formed in such a way that one of the electrodes (131) is designed to taper monotonically within the other of the electrodes (132) into a tip, or 2) wherein the at least two electrodes (131, 132)
between which the non-homogeneous microwave field is formed extend into the interior of the container (120) in order to be enclosed in the interior by the biological cells (220) and/or the macromolecules.

2. Microwave porator (100) according to claim 1, wherein the gap (135) has a mean width in a range between 10 µm and 500 µm.

3. Microwave porator (100) according to claim 2, wherein the gap (135) between the at least two electrodes (131, 132) has a shape in order to achieve impedance matching when the microwave field is introduced and thus to suppress reflections when the microwave field is fed in.

4. Microwave porator (100) according to any of the preceding claims, wherein the electrode structure (130) is formed in the form of a first and a second nail bed, such that nails of the first and the second nail bed are arranged adjacently to one another or so as to be slidable into one another.

5. Microwave porator (100) according to any of the preceding claims, wherein the container (120) has a displaceable plunger (140) and one of the at least two electrodes (131) is formed on an edge region of the container and the second of the at least two electrodes (132) is formed on the plunger (140) such that, when the plunger (140) is displaced, a spacing between the two electrodes (131, 132) changes and the biological cells (220) can be introduced between the plunger (140) and the edge region.

6. Microwave porator (100) according to any of the preceding claims, wherein at least a region of the container (120) has a window (129) to facilitate optical analysis of progress in the treatment of the biological cells (220).

7. Microwave porator (100) according to any of the preceding claims, wherein the container (120) comprises a temperature sensor (150) to facilitate sensing the temperature of the biological cells (220) during treatment.

8. Microwave porator (100) according to any of the preceding claims, further comprising a control unit (200), which is designed to apply the microwave field to the supply line (110) or to perform impedance spectroscopy for determining the success of introducing the macromolecules into the biological cells (220), in which impedance spectroscopy an impedance measurement of the biological cells (220) takes place by means of a sensor alternating field.

9. Microwave porator (100) according to claim 8, wherein the control unit (200) is designed to perform impedance spectroscopy using the electrode structure (130) or using further measuring electrodes.

10. Microwave porator (100) according to any of the preceding claims, wherein the electrode structure (130) extends through a container wall and has contacts on an outer region of the container (120), to which contacts the supply line (110) can be contacted.

11. Microwave porator (100) according to any of the preceding claims, wherein the container (120) is part of a plurality of containers (121, 122, ...) which are arranged displaceably with respect to one another and which each have the at least two electrodes (131, 132), such that contacting of the supply line (110) via the respective electrodes in the containers (121, 122, ...) takes place by displacement of the containers (121, 122, ...).

12. Method for treating biological cells (220) using a microwave porator (100) according to any of the preceding claims.

## Revendications

1. Dispositif de formation de pores par micro-ondes (100) destiné à introduire des macromolécules dans des cellules biologiques (220), comportant les caractéristiques suivantes :
une alimentation (110) pour un champ de micro-ondes ;
un récipient (120) pour les cellules biologiques (220) et les macromolécules ; et
une structure d'électrodes (130) comportant au moins deux électrodes (131, 132)
qui sont réalisées au moins partiellement dans le récipient (120) et qui sont reliées ou peuvent être reliées à l'alimentation (110) pour exposer les cellules biologiques (220) au champ de micro-ondes,
la structure d'électrodes (130) s'étendant le long d'un bord ou dans un espace interne du récipient et présentant une forme telle qu'un champ de micro-ondes inhomogène se forme entre les au moins deux électrodes (131, 132), 1) une fente (135) étant formée entre les au moins deux électrodes, laquelle fente 1a) s'étend sous forme de méandres ou 1b) est conçue de manière telle que l'une des électrodes (131) au sein des autres électrodes (132) est réalisée de manière uniformément effilée en pointe, ou 2) les au moins deux électrodes (131, 132),
entre lesquelles le champ de micro-ondes inhomogène se forme, s'étendant dans l'espace interne du récipient (120) pour être entourées dans l'espace interne par les cellules biologiques (220) et/ou les macromolécules.

2. Dispositif de formation de pores par micro-ondes (100) selon la revendication 1, la fente (135) présentant une largeur moyenne dans une plage comprise entre 10 µm et 500 µm.

3. Dispositif de formation de pores par micro-ondes (100) selon la revendication 2, la fente (135) entre les au moins deux électrodes (131, 132) présentant une forme pour obtenir une adaptation d'impédance lors de l'introduction du champ de micro-ondes et ainsi supprimer des réflexions lors d'une injection du champ de micro-ondes.

4. Dispositif de formation de pores par micro-ondes (100) selon l'une des revendications précédentes, la structure d'électrodes (130) étant formée sous forme d'un premier et d'un second lit de clous de manière telle que les clous du premier et du second lit de clous sont disposés les uns à côté des autres ou de manière à pouvoir s'emboîter les uns dans les autres.

5. Dispositif de formation de pores par micro-ondes (100) selon l'une des revendications précédentes, le récipient (120) présentant un piston (140) mobile et l'une des au moins deux électrodes (131) étant réalisée au niveau d'une zone de bord du récipient et la seconde des au moins deux électrodes (132) étant réalisée sur le piston (140) de manière telle que, lors d'un déplacement du piston (140), une distance entre les deux électrodes (131, 132) se modifie et les cellules biologiques (220) peuvent être introduites entre le piston (140) et la zone de bord.

6. Dispositif de formation de pores par micro-ondes (100) selon l'une des revendications précédentes, au moins une zone du récipient (120) présentant une fenêtre (129) pour permettre une analyse optique concernant une progression lors du traitement des cellules biologiques (220).

7. Dispositif de formation de pores par micro-ondes (100) selon l'une des revendications précédentes, le récipient (120) présentant un capteur de température (150) pour permettre une détection de température des cellules biologiques (220) pendant le traitement.

8. Dispositif de formation de pores par micro-ondes (100) selon l'une des revendications précédentes, lequel comprend en outre une unité de commande (200) qui est configurée pour appliquer le champ de micro-ondes à l'alimentation (110) ou pour réaliser une spectroscopie d'impédance pour déterminer le résultat de l'introduction des macromolécules dans les cellules biologiques (220), lors de laquelle spectroscopie une mesure d'impédance des cellules biologiques (220) est effectuée au moyen d'un champ alternatif de capteur.

9. Dispositif de formation de pores par micro-ondes (100) selon la revendication 8, l'unité de commande (200) étant configurée pour réaliser la spectroscopie d'impédance à l'aide de la structure d'électrodes (130) ou à l'aide d'autres électrodes de mesure.

10. Dispositif de formation de pores par micro-ondes (100) selon l'une des revendications précédentes, dans lequel la structure d'électrodes (130) s'étend à travers une paroi de récipient et présente, sur une zone externe du récipient (120), des contacts avec lesquels l'alimentation (110) peut être mise en contact.

11. Dispositif de formation de pores par micro-ondes (100) selon l'une des revendications précédentes, le récipient (120) faisant partie d'une pluralité de récipients (121, 122, ...) qui sont disposés mobiles les uns par rapport aux autres et qui présentent respectivement les au moins deux électrodes (131, 132) de manière telle que, suite à un déplacement des récipients (121, 122, ...), une mise en contact de l'alimentation (110) est effectuée par les électrodes respectives dans les récipients (121, 122, ...).

12. Procédé permettant le traitement de cellules biologiques (220) à l'aide d'un dispositif de formation de pores par micro-ondes (100) selon l'une des revendications précédentes.
